# EUROPEAN PATENT SPECIFICATION

(11) **EP 2 243 465 B1**
(45) Date of publication and mention of the grant of the patent: **20.01.2016**
(21) Application number: 09712714.6
(22) Date of filing: 23.01.2009
(51) Int. Cl.: A61K 8/27, A61K 8/29, A61K 8/41, A61K 8/46, A61K 8/893, A61K 8/894, A61K 8/06, A61Q 17/04

(54) **WATER-IN-OIL EMULSION TYPE SUNSCREEN COSMETIC**
SONNENSCHUTZ-KOSMETIKARTIKEL DER ART WASSER-IN-ÖL-EMULSION
PRODUIT COSMÉTIQUE ÉCRAN SOLAIRE DU TYPE ÉMULSION EAU-DANS-HUILE

(30) Priority: 19.02.2008 JP 2008036798
(43) Date of publication of application: 27.10.2010
(73) Proprietor: Shiseido Company, Ltd., Chuo-ku Tokyo 104-8010 (JP)
(72) Inventor: IKEBE, Yosuke, Yokohama-shi Kanagawa 224-8558 (JP); YAMAGUCHI, Kazuhiro, Yokohama-shi Kanagawa 224-8558 (JP)
(74) Representative: Ter Meer Steinmeister & Partner
(86) International application number: PCT/JP2009/000257
(87) International publication number: WO 2009/104353

(56) References cited:
- EP-A2- 2 002 822
- WO-A1-03/030856
- WO-A1-2007/000316
- WO-A2-2007/135196
- WO-A2-2007/135196
- JP-A- 8 026 972
- JP-A- 10 175 839
- JP-A- 11 292 748
- JP-A- 2005 263 794
- JP-A- 2007 131 612
- DATABASE GNPD [Online] MINTEL; 2 June 2008 (2008-06-02), "Monoi Lotion", XP002719378, Database accession no. 937331

## Description

### TECHNICAL FIELD

The present invention relates to a water-in-oil emulsified sunscreen cosmetic. More specifically, it relates to a water-in-oil emulsified sunscreen cosmetic that has superior ultraviolet protection, causes no precipitation of the ultraviolet absorbent, and has superior stability of the base agent.

### BACKGROUND ART

Ultraviolet absorbents and/or ultraviolet scattering agents (zinc oxide, titanium oxide, etc.) are added to sunscreen cosmetics in order to block ultraviolet irradiation on the skin to achieve a high SPF (Sun Protection Factor) (refer to Patent Citations 1 and 2, for example).

One of the ultraviolet absorbents added to sunscreen cosmetics is octocrylene. Octocrylene is an ultraviolet absorbent; when used with hydrophobicized ultraviolet scattering agents (zinc oxide, titanium dioxide, etc.), it generates an offensive odor over time. To solve this problem, Patent Citation 3 discloses a water-in-oil emulsified sunscreen cosmetic comprising (a) 0.2-10 wt% of octocrylene, (b) 0.2-30 wt% of hydrophobicized titanium dioxide and/or zinc oxide, (c) 0.02-8 wt% of phenylbenzimidazole sulfonic acid, and (d) a neutralizing agent for said ingredient (c). Triethanolamine is listed as the most preferable neutralizing salt (paragraph 0023).

Patent Citation 4 discloses a sunscreen cosmetic that contains, as ultraviolet absorbents, 1,4-dihydropyridine and 1,4-dihydropyran. In addition phenylbenzimidazole sulfonic acid, aminomethyl propanol 1 and such are among those listed as the various potential ingredients that can be added to this sunscreen cosmetic. However, there is no description of directly connecting these two combinations.
Patent Citation 1: Japanese Patent Laid-Open H10-120543 bulletin
Patent Citation 2: Japanese Patent Laid-Open 2002-521417 bulletin
Patent Citation 3: Japanese Patent Laid-Open 2007-217379 bulletin
Patent Citation 4: Japanese Patent Laid-Open 2005-518425 bulletin

### DISCLOSURE OF INVENTION

### TECHNICAL PROBLEM

The inventors of the present patent application took the aforementioned Patent Citation 3 into account and, for the purpose of developing a new sunscreen cosmetic, were looking into developing a sunscreen cosmetic that contains (a) dimethicodiethylbenzalmalonate and (b) phenylbenzimidazole sulfonic acid. However, when triethanolamine, which was described as the most preferable neutralizing agent in Patent Citation 3, was used, there was an unexpected problem in that the phenylbenzimidazole sulfonic acid crystallized.

The present invention is an invention achieved as a result of earnest research aiming to solve such a problem; the inventors discovered that a sunscreen cosmetic containing (a) dimethicodiethylbenzalmalonate and (b) phenylbenzimidazole sulfonic acid that used aminomethyl propanol 1 instead of triethanolamine, which was specifically recommended in the aforementioned Patent Citation 3, and had a ratio of the water phase in the water-in-oil emulsified sunscreen cosmetic of 40 wt%, unexpectedly did not cause phenylbenzimidazole sulfonic acid to crystallize, which led to a stable sunscreen cosmetic of a water-in-oil emulsified composition, thus completing the present invention.

The object of the present invention is to provide a water-in-oil emulsified sunscreen cosmetic containing (a) dimethicodiethylbenzalmalonate and (b) phenylbenzimidazole sulfonic acid, said cosmetic causing no crystallization of phenylbenzimidazole sulfonic acid and being very stable over time.

### TECHNICAL SOLUTION

That is, the present invention provides a water-in-oil emulsified sunscreen cosmetic comprising (a) 0.1-10 wt% of dimethicodiethylbenzalmalonate, (b) 0.02-8 wt % of phenylbenzimidazole sulfonic acid, and (c) a neutralizing agent for said ingredient (b), said cosmetic having a water phase content of 40 wt% or less.

Also, the present invention provides the aforementioned water-in-oil emulsified sunscreen cosmetic wherein the blend ratio of ingredient (b) is 0.5-4 wt%.

Also, the present invention provides the aforementioned water-in-oil emulsified sunscreen cosmetic wherein ingredient (c) is contained in the amount of 0.01-3 wt%.

Also, the present invention provides the aforementioned water-in-oil emulsified sunscreen cosmetic wherein ingredient (c) is aminomethyl propanol.

Also, the present invention provides the aforementioned water-in-oil emulsified sunscreen cosmetic that additionally contains (d) a silicone type surfactant in the amount of 0.01-20 wt%.

Also, the present invention further provides the aforementioned water-in-oil emulsified sunscreen cosmetic that additionally contains (e) hydrophobicized titanium dioxide and/or zinc oxide in the amount of 10.1-25 wt%.

### ADVANTAGEOUS EFFECTS

A water-in-oil emulsified sunscreen cosmetic containing (a) dimethicodiethylbenzalmalonate and (b) phenylbenzimidazole sulfonic acid, said cosmetic causing no crystallization of phenylbenzimidazole sulfonic acid and being very stable over time, can be provided.

### BEST MODE FOR CARRYING OUT THE INVENTION

The water-in-oil emulsified sunscreen cosmetic of the present invention is described in detail below.

### "(a) Dimethicodiethylbenzalmalonate"

Ingredient (a), dimethicodiethylbenzalmalonate, used in the present invention is a prior art ultraviolet absorbent; it is commercially available as, for example, "Parsol SLX" (from DSM Nutrition Japan, Ltd.), which can be used preferably.

The blend ratio of ingredient (a) is 0.1-10 wt%, preferably 2.5-7.5 wt%, of the total amount of the water-in-oil emulsified sunscreen cosmetic.

If the blend ratio is outside of the aforementioned range, then the effect of the present invention may not be manifested sufficiently.

### "(b) Phenylbenzimidazole sulfonic acid"

Ingredient (b), phenylbenzimidazole sulfonic acid, used in the present invention is a water soluble ultraviolet absorbent; it is commercially available as, for example, "Neo Heliopan Hydro" (from Symrise, Ltd.) and "Eusolex 232" (from Merck, Ltd.), which can be used preferably.

The blend ratio of ingredient (b) is 0.02-8 wt%, preferably 0.5-4 wt%, more preferably 1-3 wt%, of the total amount of the water-in-oil emulsified sunscreen cosmetic.

If the blend ratio is outside of the aforementioned range, then the effect of the present invention may not be manifested sufficiently.

### "(c) Neutralizing agent for said ingredient (b)"

Ingredient (c) used in the present invention is a neutralizing agent to neutralize the aforementioned ingredient (b). Aminomethyl propanol is used.

The blend ratio of ingredient (c) is not limited in particular as long as it is sufficient to neutralize ingredient (b). The blend ratio of ingredient (c) is preferably 0.01-3 wt % of the total amount of the sunscreen cosmetic.

### "(d) Silicone type surfactant"

In the present invention, it is preferable to add (d) a silicone type surfactant as an emulsifier. Selection of the silicone type surfactant is not limited in particular as long as it can be used in a water-in-oil emulsified system. Examples include poly (oxyethylene/oxypropylene) methylpolysiloxane copolymer, polyoxyethylene methylpolysiloxane copolymer, branched-silicone-chain type methylpolysiloxane copolymer, branched-alkyl-chain type polyoxyethylene methylpolysiloxane copolymer, branched-alkyl-chain/silicone-chain type polyoxyethylene methylpolysiloxane copolymer, cross-linked polyoxyethylene methylpolysiloxane, alkyl-group-containing cross-linked polyoxyethylene methylpolysiloxane, branched-polyglycerin-modified silicone, cross-linked-polyglycerin-modified silicone, alkyl group-containing cross-linked-polyglycerin-modified silicone, and alkyl group branched-polyglycerin-modified silicone.

### Examples of the poly

(oxyethylene/oxypropylene) methylpolysiloxane copolymer include PEG/PPG-20/22 butylether dimethicone ("KF-6012" ; from Shin-Etsu Chemical Co., Ltd.), PEG/PPG-20/20 dimethicone ("BY22-008M" ; from Dow Corning Toray Silicone Company Ltd.), Lauryl PEG/PPG-18 methicone ("5200 Formulation Aid"; from Dow Corning Toray Company Ltd.), PEG/PPG-19/19 dimethicone ("5330 Fluid"; from Dow Corning Toray Company Ltd.), and PEG/PPG-15/15 dimethicone ("5330 Fluid"; from Dow Corning Toray Company Ltd.).

Examples of the polyoxyethylene methylpolysiloxane copolymer include PEG-11 methyl ether dimethicone ("KF6011" ; from Shin-Etsu Chemical Co., Ltd.), PEG-9 dimethicone ("KF-6013" ; from Shin-Etsu Chemical Co., Ltd.), PEG-3 ("KF-6015" ; from Shin-Etsu Chemical Co., Ltd.), PEG-9 methyl ether dimethicone ("KF-6016" ; from Shin-Etsu Chemical Co., Ltd.), PEG-10 dimethicone ("KF-6017" ; from Shin-Etsu Chemical Co., Ltd.), PEG-11 methyl ether dimethicone ("KF6018" ; from Shin-Etsu Chemical Co., Ltd.), PEG-9 dimethicone ("KF-6019" ; from Shin-Etsu Chemical Co., Ltd.), and PEG-12 dimethicone ("SH3771M" , "SH3772M" , "SH3773M" , "SH3775M" ; from Dow Corning Toray Company Ltd.

Examples of the branched-silicone-chain type methylpolysiloxane copolymer include PEG-9 polydimethylsiloxyethyl dimethicone ("KF-6028" ; from Shin-Etsu Chemical Co., Ltd.).

Examples of the branched-alkyl-chain type polyoxyethylene methylpolysiloxane copolymer include PEG/PPG-10/3 oleyl ether dimethicone ("KF-6026" ; from Shin-Etsu Chemical Co., Ltd.).

Examples of the branched-alkyl-chain/silicone-chain type polyoxyethylene methylpolysiloxane copolymer include lauryl PEG-9 polydimethylsiloxyethyl dimethicone ("KF-6038" ; from Shin-Etsu Chemical Co., Ltd.).

Examples of the cross-linked polyoxyethylene methylpolysiloxane include dimethicone (dimethicone/(PEG-10/15)) crosspolymer ("KSG-210" ; from Shin-Etsu Chemical Co., Ltd.) and cyclomethicone/PEG-12 dimethicone/dimethicone crosspolymer ("9011 silicone elastomer blend" ; from Dow Corning Toray Silicone Company Ltd.).

Examples of the alkyl-group-containing cross-linked polyoxyethylene methylpolysiloxane include mineral oil/PEG-15 lauryl dimethicone crosspolymer ("KSG-310" ; from Shin-Etsu Chemical Co., Ltd.), isododecane/PEG-15 lauryl dimethicone crosspolymer ("KSG-320" ; from Shin-Etsu Chemical Co., Ltd.), trioctanoin/PEG-15 lauryl dimethicone crosspolymer ("KSG-330" ; from Shin-Etsu Chemical Co., Ltd.), and squalane/PEG-15 lauryl dimethicone crosspolymer/PEG-10 lauryl dimethicone crosspolymer ("KSG-340" ; from Shin-Etsu Chemical Co., Ltd.).

Examples of the branched-polyglycerin-modified silicone include polyglyceryl-3 dicyclohexane dimethicone ("KF-6100" ; from Shin-Etsu Chemical Co., Ltd.) and polyglyceryl-3 polydimethylsiloxy dimethicone ("KF-6104" ; from Shin-Etsu Chemical Co., Ltd.).

Examples of the cross-linked polyglycerin-modified silicone include dimethicone/(dimethicone/polyglycerin-3) crosspolymer ("KSG-710" ; from Shin-Etsu Chemical Co., Ltd.).

Examples of the alkyl-group-containing cross-linked polyglycerin-modified silicone include mineral oil/(lauryl dimethicone/polyglycerin 3) crosspolymer ("KSG-810" ; from Shin-Etsu Chemical Co., Ltd.), isododecane/(lauryl dimethicone/polyglycerin 3) crosspolymer ("KSG-820" ; from Shin-Etsu Chemical Co., Ltd.), trioctanoin/(lauryl dimethicone/polyglycerin 3) crosspolymer ("KSG-830" ; from Shin-Etsu Chemical Co., Ltd.), and squalane/(lauryl dimethicone/polyglycerin 3) crosspolymer ("KSG-840" ; from Shin-Etsu Chemical Co., Ltd.).

Examples of the alkyl group branched-polyglycerin-modified silicone include lauryl polyglyceryl-3 polydimethylsiloxyethyl dimethicone ("KF-6105" ; from Shin-Etsu Chemical Co., Ltd.).

Of those mentioned above, polyoxyethylene methylpolysiloxane copolymer, poly (oxyethylene/oxypropylene) methylpolysiloxane copolymer, branched-silicone-chain type methylpolysiloxane copolymer, and branched-alkyl-chain/silicone-chain type polyoxyethylene methylpolysiloxane copolymer are used preferably.

The lower limit of the blend ratio of ingredient (d) is preferably 0.01 wt% or more, more preferably 0.1 wt% or more, even more preferably 0.5 wt% or more, of the total amount of the water-in-oil emulsified sunscreen cosmetic. Also, the upper limit should preferably be 20 wt% or less, more preferably 10 wt% or less.

If the blend ratio is less than 0.01 wt%, then the stability of the emulsified composition may worsen. If the blend ratio is significantly over 20 wt%, then the sensation during use may worsen due to stickiness. The blend ratio is determined appropriately from the combination of the aforementioned lower limit and upper limit.

A preferable blend ratio is 0.01-20 wt%, more preferably 0.05-10 wt%, of the total amount of the water-in-oil emulsified sunscreen cosmetic. If the stability of the emulsified composition has a priority, then 0.5-10 wt% is preferable.

### "(e) Hydrophobicized titanium dioxide and/or zinc oxide"

In the present invention, it is preferable to add hydrophobicized titanium oxide and/or zinc oxide as ingredient (e). These are ultraviolet scattering agents; after a hydrophobicizing treatment they can be efficiently dispersed in the oil phase (outer phase) to increase the ultraviolet prevention effect of the present invention.

In terms of the ultraviolet scattering effect, titanium dioxide and zinc oxide are preferably in the powder form prepared as fine particles. Preferable examples of the fine particle titanium dioxide include those having an average primary particle size of 30 nm or less, more preferably those having an average primary particle size of 20 nm or less. Examples of the fine particle zinc oxide include those having an average primary particle size of 40 nm or less, more preferably those having an average primary particle size of 30 nm or less. However, they are not limited to those having these particle sizes.

The method of hydrophobicizing is not limited in particular; the treatment can be done with a prior art method. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkali earth metal salt of said fatty acid is used, and a fluorine treatment in which diethanolamine perfluoroalkylphosphate, perfluoroalkyltrimethoxysilane, etc. are used.

Examples of the hydrophobicized titanium dioxide include "TTO-S-4" , "TTO-V-4" (both from Ishihara Sangyo Kaisha, Ltd.), "MT-100TV" , and "MT-014V" (both from Tayca Corporation).

Examples of the hydrophobicized zinc oxide include "FZO-50" (from Ishihara Sangyo Kaisha, Ltd.), "MZ-700" (from Tayca Corporation), and "Z-Cote HP-1" (from BASF).

In the present invention, commercially available products of these can be preferably used.

The blend ratio of the ingredient (e) is 10.1-25 wt% of the total amount of the water-in-oil emulsified sunscreen cosmetic. Outside of this range may not be preferable in terms of the ultraviolet scattering effect and the sensation during use (squeakiness and such) due to the addition of the powder.

If the blend ratio of ingredient (e) is 10.1 wt% or more, then a very high ultraviolet scattering effect can be obtained, but usually the occurrence of an offensive odor due to the ultraviolet absorbent and these powders is expected. However, in the present invention, it is possible to prevent/suppress the offensive odor in a stable manner.

In the present invention, it is essential that the blend ratio of the water phase (inner phase) be 40 wt% or less of the total amount of the water-in-oil emulsified sunscreen cosmetic. The preferable range is 10-40 wt%.

The ingredient of the water phase is water; however, the 40 wt% is a total of water and the water based ingredients dissolved in water (not including the surfactant). Ingredients (b) and (c) are included, but ingredients (a), (d), and (e) are not included.

On the other hand, the oil phase (outer phase) is preferably 60-85 wt% of the total amount of the water-in-oil emulsified sunscreen cosmetic. Selection of the oil component that constitutes the oil phase is not limited in particular.

In addition to the aforementioned ingredients, other ingredients usually used in cosmetics can be added as necessary into the water-in-oil emulsified sunscreen cosmetic of the present invention as long as the object and/or effect of the present invention is not adversely affected. Examples of such ingredients include water soluble polymers, oil soluble polymers, polymer powders, emulsifiers (other than the aforementioned ingredient (d)), waxes, alcohols, liquid fats and oils, ester oils, hydrocarbon oils, silicone oils, fatty acids, higher alcohols, fatty acid esters, drugs, ultraviolet absorbents (other than the aforementioned ingredient (e)), ultraviolet scattering agents (other than the aforementioned ingredient (a) and ingredient (b)), and organic modified-clay minerals. Details are illustrated below.

Examples of the water soluble polymers include homopolymer or copolymer of 2-acrylamide-2-methylpropanesulfonic acid (hereafter abbreviated as "AMPS"). Examples of the copolymer include copolymers with vinylpyrrolidone, acrylic acid amide, sodium acrylate, and hydroxyethyl acrylate, etc. Therefore, examples include AMPS homopolymer, vinylpyrrolidone/AMPS copolymer, dimethylacrylamide/AMPS copolymer, acrylic acid amide/AMPS copolymer, and sodium acrylate/AMPS copolymer.

Examples further include carboxyvinyl polymer, ammonium polyacrylate, sodium polyacrylate, sodium acrylate/alkyl acrylate/sodium methacrylate/alkyl methacrylate copolymer, carrageenan, pectin, mannan, curdlan, chondroitin sulfuric acid, starch, glycogen, gum arabic, sodium hyaluronate, traganth gum, xanthan gum, mucoitin sulfuric acid, hydroxyethyl guar gum, carboxymethyl guar gum, guar gum, dextran, kerato sulfate, locustbean gum, succinoglucane, chitin, chitosan, carboxymethyl chitin, and agar.

Examples of the oil soluble polymers include trimethylsiloxysilicate, alkyl modified silicone, and polyamide modified silicone.

Examples of the polymer powders include dimethicone crosspolymer, (dimethicone/vinyl dimethicone) crosspolymer, polymethylsilsesquioxane, polyethylene, and methyl polymethacrylate.

Examples of the waxes include honeybee wax, candelilla wax, carnauba wax, lanolin, liquid lanolin, and jojoba wax.

Examples of the emulsifiers include glycerin fatty acid esters, polyglycerin fatty acid esters, polyoxyethylene glycerin fatty acid esters, sorbitan fatty acid esters, and polyoxyethylene sorbitan fatty acid esters.

Examples of the alcohols include lower alcohols such as ethanol and isopropanol, higher alcohols such as isostearyl alcohol, octyldodecanol, and hexyldecanol, and polyhydric alcohols such as ethylene glycol, propylene glycol, 1,3-butylene glycol, dipropylene glycol, and polybutylene glycol.

Examples of the liquid fats and oils include avocado oil, tsubaki oil, turtle fatty acid, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japan gimlet oil, jojoba oil, germ oil, and triglycerin.

Examples of the ester oils include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, iso cetyl stearate, iso cetyl isostearate, cholesteryl 12-hydroxystearate, di-2-ethylene glycol ethylhexanoate, dipentaerythritol fatty acid ester, n-alkylene glycol monoisostearate, neopentyl glycol dicaprate, diisostearyl malate, glyceryl di-2-heptylundecanoate, trimethylolpropane tri-2-ethyl hexanoate, trimethylolpropane triisostearate, tetra-2-pentaerythritol ethylhexanoate, glycerin tri2-ethylhexanoate, glyceryl trioctanoate, glycerin triisopalmitate, trimethylolpropane triisostearate, cetyl 2-ethyl hexanoate, 2-ethylhexyl palmitate, glycerin trimyristate, tri-2-heptyl undecanoic acid glyceride, methyl castor oil fatty acid, oleyl oleate, aceto glyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the hydrocarbon oils include liquid petrolatum, ozocerite, squalane, pristane, paraffin, ceresin, squalene, petrolatum, microcrystalline wax, polyethylene wax, and Fischer-Tropsch wax.

Examples of the silicone oils include dimethylpolysiloxane, octamethyl siloxane, decamethyltetrasiloxane, methyl hydrogen polysiloxane, methylphenyl polysiloxane, hexamethyl cyclotrisiloxane, octamethyl cyclotetrasiloxane, and decamethyl cyclopentasiloxane.

Examples of the fatty acids include lauric acid, myristic acid, palmitic acid, stearic acid, behenic acid, and arachidonic acid.

Examples of the higher alcohols include lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, koryanyl alcohol, myricyl alcohol, lacceryl alcohol, elaidyl alcohol, isostearyl glyceryl ether, octyl alcohol, triacontyl alcohol, serachyl alcohol, cetostearyl alcohol, oleyl alcohol, lanolin alcohol, hydrogenated lanolin alcohol, hexyl decanol, and octyl decanol.

Examples of the fatty acid esters include myristyl myristate, cetyl palmitate, choresteryl stearate, and bees wax fatty acid 2-octyldedecyl ester.

Examples of the drugs include salts of L-ascorbic acid and its derivatives, glycyrrhizic acid and its derivatives such as dipotassium glycyrrhizate and monoammonium glycyrrhizate, glycyrrhetinic acid and its derivatives such as stearyl glycyrrhetinate, allantoin, salts of tranexamic acid and its derivatives, salts of alkoxysalicylic acid and its derivatives, salts of glutathione and its derivatives, allantoin, and azulene.

Examples of the ultraviolet absorbents other than the aforementioned ingredients (a) and (b) include cinnamic acid derivatives such as ethylhexyl methoxycinnamate, isopropyl methoxycinnamate, and isoamyl methoxycinnamate; para-aminobenzoic acid (hereafter abbreviated as PABA) derivatives such as PABA, ethyl PABA, ethyl-dihydroxypropyl 1 PABA, ethylhexyl-dimethyl 1 PABA, and glyceryl PABA; salicylic acid derivatives such as homosalate, ethylhexyl salicylate, dipropylene glycol salicylate, and TEA salicylate; benzophenone derivatives such as benzophenone-1, benzophenone-2, benzophenone-3 or oxybenzone, benzophenone-4, benzophenone-5, benzophenone-6, benzophenone-8, benzophenone-9, and benzophenone-12; benzylidene camphor derivatives such as 3-benzylidene camphor, 4-methylbenzylidene camphor, benzylidene camphor sulfonic acid, camphor benzalkonium methosulfate, terephthalylidene camphor sulfonic acid, and polyacrylamide methylbenzylidene camphor; triazine derivatives such as anisotriazine, ethylhexyl triazone, diethylhexyl butamido triazone, and 2,4,6-tris (diisobutyl-4'-aminobenzalmalonate)-s-triazine; phenylbenzimidazole derivatives such as disodium phenyldibenzimidazole tetrasulfonate; phenylbenzotriazole derivatives such as drometrizole, trisiloxane and methylenebis (benzotriazoryl tetrabutylphenol); anthranyl derivatives such as mentyl anthranilate; imidazoline derivatives such as ethylhexyldimethoxybenzylidene dioxoimidazoline propionate; benzalmalonate derivatives such as polyorganosiloxane having benzalmalonate functional groups; 4,4-diarylbutadiene derivatives such as 1,1-dicaroboxy (2,2'-dimethylpropyl)-4,4-diphenylbutadiene.

Examples of ultraviolet scattering agents other than the aforementioned ingredient (e) include hydrophobicized inorganic pigments such as kaolin and calcium carbonate.

Examples of the organic modified clay minerals include quaternary ammonium salt type cation-modified clay minerals.

The product forms of the water-in-oil emulsified sunscreen cosmetic of the present invention include emulsions and creams. These products can be prepared by an ordinary method using the aforementioned essential ingredients and other ingredients usually contained in cosmetics.

### EXAMPLES

Next, the present invention is described in detail by referring to Examples. The present invention is not limited to the following Examples. The blend ratios are all in wt% units.

### "Example 1, Comparative examples 1-4"

Sunscreen cosmetics were prepared based on the recipes shown in the following Table 1. Specifically, (9) <part B> was added to (1)-(8) <part A> and homogeneously dispersed, to which (10)-(12) <part C> was added and homogeneously dispersed, and then (13)-(20) <part D> was gradually added and emulsified to obtain a water-in-oil emulsified sunscreen cosmetic.

The water phase of Example 1 and Comparative examples 1-4 is 30. 9-31. 15 wt%.

### <Whether crystallization occurs or not>

The obtained sunscreen cosmetics (samples) were evaluated based on the following to check whether the phenylbenzimidazole sulfonic acid crystallized or not. The results are shown in Table 1.

### <Test method>

Each sample was left at rest for one month while the temperature was cycled between 0° C and 40° C every hour and observed with a microscope (BX-50-33PHD x400 from Olympus) to evaluate whether or not there were crystals by using the following evaluation criteria.

### <Evaluation criteria>

Crystals are observed: Yes
Crystals are not observed: No

### <Stability in terms of preventing/suppressing an offensive odor>

The obtained sunscreen cosmetics (samples) were evaluated based on the following for the offensive odor prevention/suppression effect. The results are shown in Table 1.

### <Test method>

Each sample was left at rest for one month in a thermostatic tank at 50° C and 10 female panelists actually used it to evaluate the odor at the time of application based on the following evaluation criteria.

### <Evaluation criteria>

⊚ : 9 or more out of 10 reported that the odor was within tolerance.
○ : 6-8 out of 10 reported that the odor was within tolerance.
Δ : 3-5 out of 10 reported that the odor was within tolerance.
X: Less than 3 out of 10 reported that the odor was within tolerance.

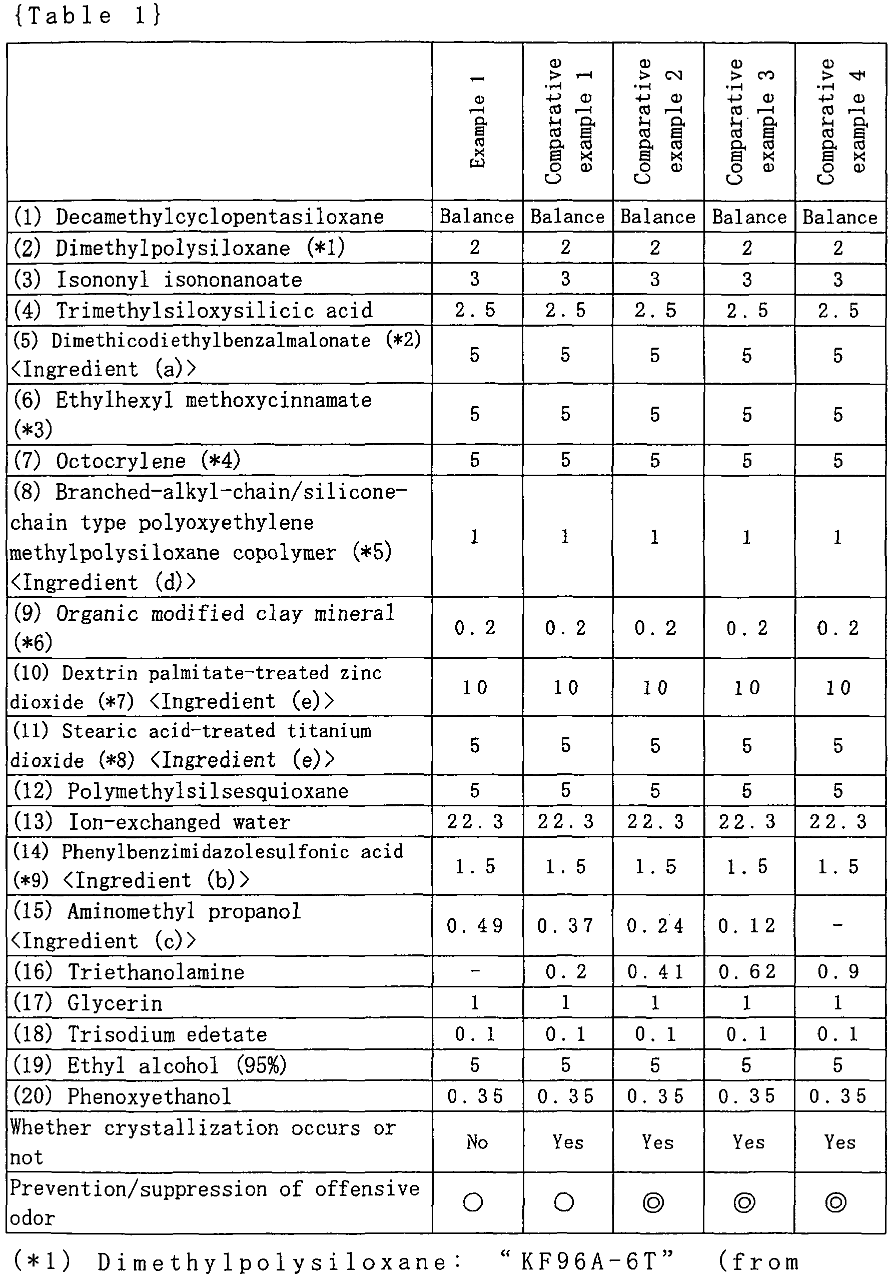

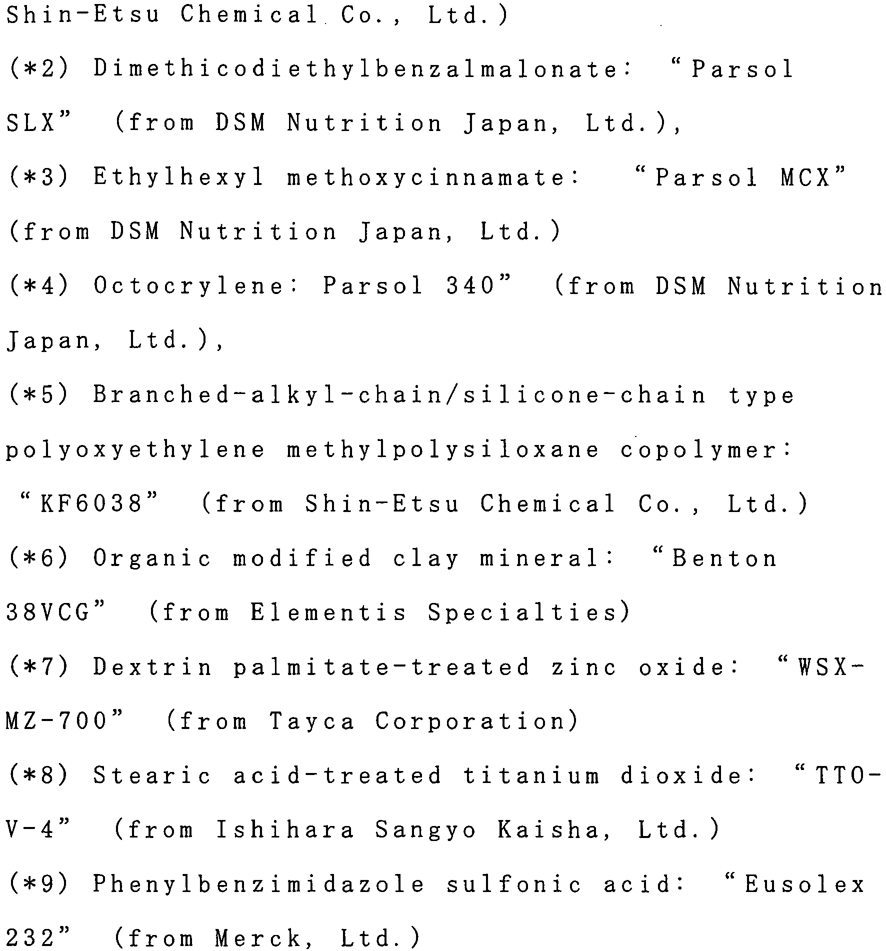

As clearly shown in Table 1, the water-in-oil emulsified sunscreen cosmetic of the present invention does not generate crystals of phenylbenzimidazole sulfonic acid even after being left at rest at 50° C, indicating good stability. It is also verified to be superior in terms of the offensive odor prevention/suppression effect.

### INDUSTRIAL APPLICABILITY

The present invention can provide a water-in-oil emulsified sunscreen cosmetic that has superior ultraviolet protection, causes no precipitation of the ultraviolet absorbent, and has superior stability of the base agent. The present invention can be used preferably as a sunscreen product in the emulsion form.

## Claims

1. A water-in-oil emulsified sunscreen cosmetic comprising (a) 0.1-10 wt% of dimethicodiethylbenzalmalonate, (b) 0.02-8 wt % of phenylbenzimidazole sulfonic acid, and (c) a neutralizing agent for said ingredient (b), said cosmetic having a water phase content of 40 wt% or less, wherein ingredient (c) is aminomethyl propanol.

2. The water-in-oil emulsified sunscreen cosmetic of claim 1 that contains ingredient (b) in the amount of 0. 5-4 wt%.

3. The water-in-oil emulsified sunscreen cosmetic of claim 1 or 2 that contains ingredient (c) in the amount of 0.01-3 wt%.

4. The water-in-oil emulsified sunscreen cosmetic of any of claims 1-3 that additionally contains (d) a silicone type surfactant in the amount of 0.01-20 wt%.

5. The water-in-oil emulsified sunscreen cosmetic of any of claims 1-4 that contains (e) hydrophobicized titanium dioxide and/or zinc oxide in the amount of 10.1-25 wt%.

## Patentansprüche

1. Wasser-in-Öl-emulgiertes Sonnenschutz-Kosmetikum, umfassend (a) 0,1-10 Gew.-% Dimethicodiethylbenzalmalonat, (b) 0,02-8 Gew.-% Phenylbenzimidazolsulfonsäure und (c) ein Neutralisierungsmittel für den Bestandteil (b), wobei das Kosmetikum einen Wasserphasengehalt von 40 Gew.-% oder weniger aufweist, wobei Bestandteil (c) Aminomethylpropanol ist.

2. Wasser-in-Öl-emulgiertes Sonnenschutz-Kosmetikum nach Anspruch 1, welches Bestandteil (b) in der Menge von 0,5-4 Gew.-% enthält.

3. Wasser-in-Öl-emulgiertes Sonnenschutz-Kosmetikum nach Anspruch 1 oder 2, welches Bestandteil (c) in der Menge von 0,01-3 Gew.-% enthält.

4. Wasser-in-Öl-emulgiertes Sonnenschutz-Kosmetikum nach mindestens einem der Ansprüche 1-3, welches zusätzlich (d) ein Tensid vom Silicontyp in der Menge von 0,01-20 Gew.-% enthält.

5. Wasser-in-Öl-emulgiertes Sonnenschutz-Kosmetikum nach mindestens einem der Ansprüche 1-4, welches (e) hydrophobisiertes Titandioxid und/oder Zinkoxid in der Menge von 10,1-25 Gew.-% enthält.

## Revendications

1. Cosmétique de type filtre de soleil sous forme d'émulsion eau dans huile comprenant (a) 0,1 à 10 % en poids de diméthicodiéthylbenzalmalonate, (b) 0,02 à 8 % en poids d'acide phénylbenzimidazole sulfonique, et (c) un agent de neutralisation pour le dit composant (b), le dit cosmétique ayant un contenu de phase aqueuse de 40 % en poids ou moins, dans lequel le composant (c) est l'aminométhyl-propanol.

2. Le cosmétique de type filtre de soleil sous forme d'émulsion eau dans huile selon la revendication 1 qui contient le composant (b) en la quantité de 0,5 à 4 % en poids.

3. Le cosmétique de type filtre de soleil sous forme d'émulsion eau dans huile selon la revendication 1 ou 2 qui contient le composant (c) en la quantité de 0,01 à 3 % en poids.

4. Le cosmétique de type filtre de soleil sous forme d'émulsion eau dans huile selon l'une quelconque des revendications 1 à 3 qui contient en plus (d) un tensioactif de type silicone en une quantité de 0,01 à 20 % en poids.

5. Le cosmétique de type filtre de soleil sous forme d'émulsion eau dans huile selon l'une quelconque des revendications 1 à 4 qui contient (e) du dioxyde de titane et/ou de l'oxyde de zinc rendu hydrophobe en la quantité de 10,1 à 25 % en poids.
